(19) Europäisches Patentamt
European Patent Office
Office européen des brevets

(11) Publication number: 0 212 726
A2

(12) # EUROPEAN PATENT APPLICATION

(21) Application number: 86201335.6

(22) Date of filing: 29.07.86

(51) Int. Cl.⁴: C 07 D 237/28, C 07 D 401/12, C 07 D 405/12, A 01 N 43/58

(30) Priority: 15.08.85 US 765630
29.08.85 US 770464

(43) Date of publication of application: 04.03.87 Bulletin 87/10

(84) Designated Contracting States: AT BE CH DE FR GB IT LI LU NL SE

(71) Applicant: SHELL INTERNATIONALE RESEARCH MAATSCHAPPIJ B.V., Carel van Bylandtlaan 30, NL-2596 HR Den Haag (NL)

(72) Inventor: Denes, Lucian Radu, 1105 Palmilla Drive, Modesto California 95356 (US)
Inventor: John, William Wynn, 3808 Atwood Drive, Modesto California 95355 (US)
Inventor: Soloway, Samuel Barney, 3401 Mansfield Lane, Modesto California 95350 (US)
Inventor: Estreicher, Herbert, 3901 Hillsboro Court, Modesto California 95352 (US)
Inventor: Zupan, Mike, 9963 Tralee Way, Elk Grove California 95624 (US)

(74) Representative: Hunter, Keith Roger Ian et al, 4 York Road, London SE1 7NA (GB)

(54) **Herbicidal cinnoline compounds.**

(57) The growth of unwanted plants is controlled by certain optionally substituted cinnoline-4-carboxylic acids and ester, amino, hydroxamic acid and hydrazide derivatives thereof, and salts and N-oxides of such compounds, The invention further includes compositions containing such compounds, the preparation of the compounds and compositions, and certain novel compounds per se.

EP 0 212 726 A2

1

K 3436 III FF

# HERBICIDAL CINNOLINE COMPOUNDS

This invention relates to the use of certain cinnoline-4-carboxylic acids and congeners thereof in controlling the growth of unwanted plants, to herbicidal compositions incorporating such compounds, to certain novel compounds *per se* and to their preparation.

Cinnoline-4-carboxylic acid and its ethyl ester are known from J. Am. Chem. Soc., 1946, 68, 1310-3. The acid currently is available commercially. The 2-N-oxide of the acid is also known (Registry number 20864-43-1) whilst J. Org. Chem., 26, 2374-8, 1961 discloses certain 4-dialkylaminoalkoxycarbonyl cinnolines. None of these compounds has been disclosed as having herbicidal properties.

UK 1594621 discloses that various 4-substituted cinnoline compounds, but not cinnoline-4-carboxylic acids or their congeners, have activity against ticks. One such compound is 4-phenyl cinnoline. One test described in UK 1594621 involved spraying the compounds onto vetch aphids located on broad bean plants trimmed to one leaf each. In the equivalent US patent, US 4125613, 4-phenyl cinnoline was erroneously identified as cinnoline-4-carboxylic acid. However, the error was apparent to the person skilled in the art at the time of grant of this US patent, since correct versions of the equivalent specifications

had already been published in other countries, and the convention document on the file of the US patent identified the compound correctly as 4-phenyl cinnoline. The error was corrected by means of a reissue procedure, US 4125613 being replaced by US reissue patent, No. Re. 31623.

According to the present invention there is provided a method of controlling the growth of unwanted plants by application of a compound of the formula:

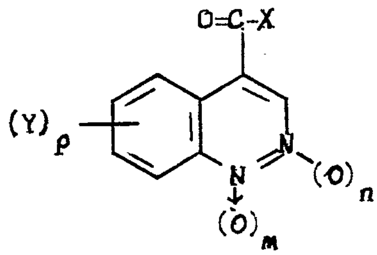

(I)

wherein m is zero or one, n is zero or one, (with the proviso that at least one of m and n is zero), p is zero, one or two, (with the proviso that when p is two, one of Y is methyl or ethyl), X is one of the moieties: $-NH_2$, $-NHNH_2$, -NHOH and -O-R, wherein R is

(a) hydrogen;

(b) alkyl of one to ten carbon atoms;

(c) alkyl or one to ten carbon atoms substituted by one of cyano, alkanoyl, carboxy, alkoxycarbonyl, aminocarbonyl, mono- and dialkylaminocarbonyl, benzyloxycarbonyl, alkylthiocarbonyl, alkenyl and alkynyl of two to four carbon atoms, cycloalkyl of three to six carbon atoms, oxiranyl dialkoxyphosphinyl, tetrahydrofuranyl, tetrahydropyranyl, dioxanyl, dioxolanyl, pyridinyl, pyridinyl-N-oxide, and optionally substituted phenyl;

(d) alkyl of two to ten carbon atoms substituted by one to three halogen atoms (bromine, chlorine, fluorine); one of alkylthio, alkanoyloxy, mono- and dialkylamino, moieties of the formula $-(-O-R^1-)_n-O-R^2$ (in which $R^1$ is alkylene of one to four carbon atoms, $R^2$ is hydrogen or alkyl of one to six

carbon atoms and n is one, two, three or four), or one to four of hydroxy and alkoxy, with the proviso that in this substituted alkyl moiety the carbon atom bonded to the indicated oxygen atom is unsubstituted ($-CH_2-$);

(e) cycloalkyl of three to six carbon atoms;

(f) optionally substituted phenyl;

(g) pyridinyl;

(h) heterocyclyl of five or six atoms containing one or two oxygen atoms bonded only to carbon therein;

and Y is

(a) alkyl of one to ten carbon atoms;

(b) alkyl of one to ten carbon atoms substituted by one of fluorine, cyano, carboxy, alkenyl of two to four carbon atoms, benzyl, alkoxycarbonyl, alkylthio, alkylsulfinyl, alkoxy and phenoxy;

and salts of those compounds wherein R is hydrogen or X is -NHOH.

In these compounds, an alkyl substituent moiety preferably has 1 to 6 carbon atoms especially 1 to 4, and may be either straight-chain or branched-chain. Suitable substituents on any phenyl moiety include mono- and polyhalo, or one or two of mono- and polyhaloalkyl, carboxy, nitro, alkoxycarbonyl, alkyl, alkoxy and alkylthio. In these substituent moieties, each alkyl moiety suitably contains from one to four carbon atoms, and is either straight-chain or branched-chain.

The contemplated salts are those of alkali metals, alkaline earth metals, amines and ammonia. Suitable amine salts are those of mono-, di- and tri-alkyl- and -alkanol- amines wherein each alkyl moiety contains up to twenty carbon atoms.

Preferably X represents a group of formula -OR, where R may be as defined above but preferably represents a hydrogen atom or a substituted or, especially, an unsubstituted alkyl group.

In a preferred subgenus of the compounds of Formula I p is one or two and a substituent Y is bonded to the carbon atom in the 8-position, since compounds of that subgenus appear to have

the widest spectrum of phytotoxic activity -- i.e., the number of different varieties of plants that are adversely affected by the compounds.

The undesired plant growth it is desired to combat is, of course, generally weed growth. The method of the invention generally involves the application of a compound as defined above to a field in which the undesired plant growth is occurring or is anticipated. The method of this invention is particularly applicable to combating weed growth amongst soya crops.

The preparation, isolation and testing of typical individual species of the compounds of Formula I are described in the examples, following. The class of compounds is further illustrated and exemplified by the following further individual species, all of which are specifically contemplated in this invention. In the interest of brevity, and clarity, and to avoid repetition of sometimes long chemical names, these species will be identified in terms of Formula I and the symbols used therein. The following species of the subgenus wherein X is $CH_3O-$, m and n each is zero:

| Species No. | Y (number indicates position on ring) |
|---|---|
| 77 | 5-methyl- |
| 78 | 8-propyl- |
| 79 | 8-butyl- |
| 80 | 8-(1,1-dimethylethyl)- |
| 81 | 8-(ethenyl)- |
| 82 | 8-(1-methylethenyl)- |
| 83 | 8-(cyanomethyl)- |
| 84 | 8-(carboxymethyl)- |
| 85 | 8-(methoxycarbonylmethyl)- |
| 86 | 8-(methylthiomethyl)- |
| 87 | 8-(methylsulphonylmethyl)- |
| 88 | 8-(methoxymethyl)- |
| 89 | 8-(phenoxymethyl)- |

| | |
|---|---|
| 90 | 8-(difluoromethyl)- |
| 91 | 8-(1-cyano-1-methylethyl)- |
| 92 | 8-(1-carboxy-1-methylethyl)- |
| 93 | 8-(1-methylthio)-1-methylethyl)- |
| 94 | 8-(1-methylsulphonyl-1-methylethyl)- |
| 95 | 5,8-dimethyl- |
| 96 | 8-cyclopropyl- |
| 97 | 8-(1-methylcyclopropyl)- |
| 98 | 5-methyl-8-(1-methylethyl)- |
| 99 | 5-(1-methylethyl-8-(methyl)- |
| 100 | 8-(1-propenyl)- |
| 101 | 8-(2-methyl-1-propenyl)- |
| 102 | 8-benzyl |
| 103 | 6,8-dimethyl- |
| 104 | 5,6-dimethyl- |
| 105 | 5,7-dimethyl- |

The following are species wherein p is one:

| Species No. | X | m | n | $(Y)_p$ (number indicates position on ring) |
|---|---|---|---|---|
| 106 | -0-2-(2-(2-methoxy-ethoxy)ethoxy)ethyl | 0 | 0 | 8-methyl |
| 107 | -O-2-(2-(2-(2-hydroxy-ethoxy)ethoxy)ethoxy)-ethyl | 0 | 0 | 8-ethyl |
| 108 | -O-(1,3-dioxolan-4-yl)methyl | 0 | 0 | 8-ethyl |
| 109 | -O-2-(2-hydroxy-1-methylethoxy)propyl | 0 | 0 | 8-ethyl |
| 110 | -O-(1,3-dioxolan-2-yl)methyl | 0 | 0 | 8-ethyl |
| 111 | -O-2-(methoxy)ethyl | 0 | 0 | 8-ethyl |
| 112 | -O-pentyl | 1 | 0 | 8-methyl |
| 113 | -O-butyl | 1 | 0 | 8-methyl |

| | | | | |
|---|---|---|---|---|
| 114 | -O-butyl | 1 | 0 | 8-ethyl |
| 115 | -O-propyl | 1 | 0 | 8-(1-methylethyl) |

The following are species of the subgenus wherein p is zero:

| Species No. | X | n | m |
|---|---|---|---|
| 116 | -O-butyl | 1 | 0 |
| 117 | -O-butyl | 0 | 1 |
| 118 | -O-pentyl | 1 | 0 |
| 119 | -O-pentyl | 0 | 1 |
| 120 | -O-hexyl | 1 | 0 |
| 121 | -O-hexyl | 0 | 1 |
| 122 | -O-(3-methylbutyl) | 1 | 0 |
| 123 | -O-(3-methylbutyl) | 0 | 1 |

There is further provided, in accordance with the present invention, a herbicidal composition comprising a compound of the general formula I, as defined above, and at least two carriers, at least one of which is a surface active agent.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium alluminium silicates, for example attapulgites and vermiculites; aluminium silicates, for example kaolinites, montomorillonites and micas; calcium carbonate; calcium sulphate; ammonium

sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example <u>p</u>-octylphenol or <u>p</u>-octylcresol, with ethylene oxide and/or propylene oxide; sulphates or sulphonates of these condensation products; alkali or alkaline earth metal salts,

preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing ½-10% w of active ingredient. Granules are usually prepared to have a size between 10 and 100 BS mesh (1.676 - 0.152 mm), and may be manufactured by agglomeration or impregnation techniques. Generally, granules will contain ½-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension conentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of

other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing herbicidal, insecticidal or fungicidal properties.

In other aspects of invention there is provided a method of preparing such a herbicidal composition by mixing a compound of formula I with a carrier, and the use of a compound or composition as defined above in combating undesired plant growth. Application to the locus may be pre-emergence or post-emergence, that is, to soil in which the seeds of the unwanted plants are present, or to the foliage of the unwanted plants. The active compound, of course, is applied in an amount sufficient to exert the desired action.

The amount of the compound of the invention to be used in combating undesired plants will naturally depend on the condition of the plants, the degree of activity desired, the formulation used, the mode of application, the climate, the season of the year, and other variables. Recommendations as to precise amounts are, therefore, not possible. In general, however, application to the locus to be protected of from 0.05 to 10.0kg per hectare of the compound of Formula I will be satisfactory, especially 0.1 to 4.0kg per hectare.

The present invention further provides novel compounds of formula I, as defined above, provided that when p and m are both 0 and X is -OR, R is not hydrogen, ethyl or di($C_{1-2}$alkyl)amino-$C_{1-2}$alkyl.

Further in accordance with the invention is a novel process for preparing the compounds of formula I, as defined above, or in any of claims 1 to 4 or 7 hereinafter, which comprises treating a corresponding (4-trichloromethyl)cinnoline with concentrated sulphuric acid, to form a (4-carboxylic acid)cinnoline of formula I, and, when an ester of formula I is required, converting said acid to the ester, and when a compound of formula I is required in which X represents the group $-NHNH_2$, treating a said acid with hydrazine and, when a compound of formula I is required in which X represents the group -NHOH, treating a said ester with hydroxylamine; and, when a compound of formula I is required in which X is a group $-NH_2$, treating a 4-carbonitrile cinnoline compound with hydroxylamine; and converting a compound thus prepared into a salt or N-oxide thereof, or into any other compound of formula I.

Preferably, the (4-trichloromethyl)cinnoline is treated with at least an equivalent amount of concentrated sulphuric acid. Particularly good results are achieved when the cinnoline is treated with at least three moles of acid per mole of the cinnoline. Preferably, somewhat more than this amount of acid is used - for example four to five moles of acid per mole of the cinnoline reagent. A larger amount of acid is not know to provide any additional advantage, and is to be avoided to minimize consumption of the acid, and to minimize the amount of water needed to quench the final acidic reaction mixture.

By concentrated sulphuric acid is meant pure sulfuric acid, and solutions of sulfuric acid in water containing at least 93% sulphuric acid. Commercially available concentrated sulfuric acids containing 95-96% sulfuric acid are preferable because they are readily available at minimum cost.

Treatment of the trichloromethylcinnoline with the acid is conveniently accomplished by slowly adding the cinnoline to the stirred acid, initially at ambient temperature. The reaction is mildly exothermic, and may be allowed to proceed without cooling while the cinnoline is being added. Thereafter, the mixture is stirred and heated at a temperature of about 125°C - 150°C for a period of time sufficient to enable the reaction to proceed essentially to completion. The reaction mixture may then be quenched with ice, then made basic with an aqueous solution of a metal base, such as sodium hydroxide, or concentrated ammonium hydroxide, and the resulting mixture may be washed with a solvent, such as methylene chloride, to remove any unreacted connoline, and filtered. The filtrate may then carefully acidified to give the acid, hydrochloric acid being a suitable acidifying agent.

It is desirable that the hydrogen chloride evolved during the reaction of the cinnoline and sulfuric acid be removed from the reaction zone essentially as it forms.

The cinnoline precursors of formula

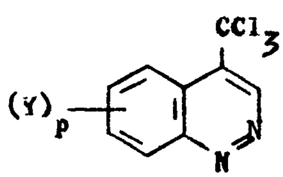

(II)

can be prepared by treating the corresponding 4-methylcinnoline with sodium hypochlorite of certain concentration in a liquid phase comprising essentially water and a polar, water-miscible inert organic liquid as co-solvent, the pH of the liquid medium being controlled within a certain range. This method is preferably conducted by contacting the 4-methyl cinnoline with the mixture of sodium hypochlorite, water and water-miscible solvent at about ambient temperature (i.e., from about 15°C to about 30°C) for sufficient time for the reaction to go to completion (ordinarily of the order of 5 to 60 hours). The

method is conveniently conducted by dissolving the 4-methylcinnoline reactant in the co-solvent, then mixing the solution with the aqueous solution of sodium hypochlorite. A concentration of sodium hypochlorite of at least eight per cent by weight, based upon the weight of the liquid phase, is required, and it is preferred that the concentration be at least ten per cent, on the same basis. A higher concentration - for example, up to fifteen per cent on the same basis, may be employed, but preferably a concentration of no more than about thirteen per cent is employed, to avoid possible side-reactions.

The aqueous liquid medium for the preparation of the compounds II comprises essentially water and a polar, water-miscible inert organic liquid as co-solvent. Examples of such liquids are dioxane, diglyme, sulfolane and dimethylformamide. At least five volumes of the liquid per one hundred volumes of water are required, but the amount of the liquid should not be such as to create a substantial amount of a second liquid phase in the reaction mixture.

The pH of the reaction mixture initially must be within the range of from about 12 to about 13.5. If an equivalent amount of chlorine is bubbled into a solution of sodium hydroxide, the resulting solution of sodium hypochlorite has a pH of about 10. Such a solution is not suitable in this method. In such a case, the pH can be be brought into the requisite range by addition of sodium hydroxide. Alternately, a suitable solution can be prepared by introducing chlorine into a solution of sodium hydroxide of such concentration that the final solution contains the required concentration of sodium hypochlorite and is within the required pH range. Solutions of sodium hypochlorite sold for use in treating water in swimming pools are prepared in this way; such usually contain about 10-12.5% sodium hypochlorite, and if they have the correct pH are suitable for use in the method. If needed, the pH of such a solution can be modified by the addition of the appropriate amount of sodium hydroxide.

A stoichiometric excess of sodium hypochlorite should be employed. At least three equivalents of sodium hypochlorite are preferably used per mole of the cinnoline, and generally an excess of the hypochlorite - up to about six equivalent per mole of cinnoline - is desirable to ensure completion of the reaction in a reasonable time. A larger excess is to be avoided, to reduce the possibility of undesirable side reactions.

The 4-(trichloromethyl)cinnoline product ordinarily precipitates from the reaction mixture and can be recovered by filtration or decantation. Water washing ordinarily gives an essentially pure product, but if necessary or desired, it can be further purified by conventional techniques.

In a further aspect the invention relates to compounds of formula II.

As described by C.M. Atkinson and J.C.E. Simpson, Journal of the Chemical Society, 1947, pages 808-812, and by T.L. Jacobs, et al., Journal of the American Chemical Society, 1946, volume 68, pages 1310-1313, the 4-methylcinnoline precursors

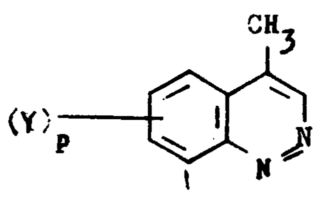

(III)

can be prepared from the appropriate anthranilic acids. The procedures involved are demonstrated in the Examples, hereinafter.

Various methods are available for preparing esters of formula I. One method is to treat the acid (or salt thereof) with oxalyl chloride, suitably in an inert organic solvent at a temperature in the range 0-100°C, and to treat the resulting acid chloride with the appropriate alcohol, conveniently in the presence of an organic solvent, at a temperature in the range 0 to 40°C. A second method is to treat the acid with carbonyldiimidazole (CDII), suitably in a polar organic solvent at a temperature in the range 0 to 40°C, and to treat the

resulting cinnoline-4-imidazolidine with the appropriate alcohol, suitably in an organic solvent at a temperature in the range 0 to 40°C, optionally in the presence of sodium imidazole. A third method is to treat the acid with a compound of formula X-Hal, where X is as defined above, but is not hydrogen, and Hal is a halogen, preferably bromine or chlorine atom, in the presence of 1,8-diazabicyclo[5.4.0]undec-7-ene (DBU) in the presence of an organic solvent, at an elevated temperature, for example 50°C to the reflux temperature.

A preferred method of preparing an ester from an acid is simply to treat the acid with the appropriate alcohol at an elevated temperature, for example 50 to 125°C, especially 70 to 90°C. This method is readily applicable without the need for isolation of the acid. Conveniently, at the end of the procedure for preparing the acid from the trichloromethylcinnoline the reaction is mixture is cooled to 20 to 40°C, the alcohol is slowly added to the mixture, and the resulting mixture is heated, with stirring, to the elevated temperature for a period of time sufficient to enable the completion of the reaction.

In this preferred esterification method an excess of alcohol over that required by the stoichiometry (one mole per mole of the cinnoline reagent used) is preferably used. Preferably, at least five moles of alcohol are used per mole of cinnoline charged, but no additional advantage is known to accrue from the use of more than about twenty moles of alcohol per mole of cinnoline.

The ester product is recovered from the crude reaction mixture by neutralizing the acid in the reaction mixture and extracting the ester therefrom. This may be accomplished by evaporating all, or most, of the excess alcohol from the crude reaction mixture, adding to the residue a suitable water-immiscible liquid in which the ester product is soluble, neutralizing the resulting mixture, separating the extract and aqueous phases and recovering the ester from the extract by

evaporating the solvent therefrom. However, it is preferred that the reaction mixture be mixed with an excess of cold water, such as ice water, and the resulting mixture be extracted with the solvent. However, to minimize the possibility that the ester will be hydrolyzed, it is desirable to quench the crude reaction mixture in a mixture of water and the solvent, then separate the extract phase - the solvent phase containing the ester product. As shown in the examples, hereinafter, the ester product is readily isolated by washing the extract to neutrality with a dilute aqueous solution of a base, drying it, then stripping it of solvent. The crude product can be purified by conventional means, such as chromatography and/or recrystallization. Suitable as the solvent for the ester is any essentially water-insoluble organic liquid in which the ester is readily soluble. Examples are methylene chloride and ethyl acetate. Sodium bicarbonate is a suitable base for effecting neutralization of the acid.

Reaction of the acid with hydrazine is preferably effected by treating the acid with CDII at ambient temperature, in an organic solvent, adding an aqueous solution for hydrazine to the reaction mixture and stirring at ambient temperature for a sufficient time for the reaction to go to completion.

Reaction of an ester with hydroxylamine, to form a hydroxamic acid, preferably employs a methyl ester, in the presence of methanol/sodium methoxide. The reaction is preferably effected at ambient temperature. The hydroxylamine is conveniently used in the form of its hydrochloride.

The reaction of hydroxylamine with 4-carbonitrile cinnolines, to prepare cinnoline-4-carboxamide compounds is preferably effected in the presence of a base and organic solvent, for example potassium hydroxide in ethanol, at ambient temperature. The hydroxylamine is preferably used in the form of its hydrochloride.

N-oxides of compounds of formula I are conveniently effected using a peracid, especially a peroxybenzoic acid, for

example m-chloroperoxybenzoic acid (MCPBA), in the presence of a halogenated alkane solvent, for example methylene chloride, to oxidise an ester of formula I. The resulting ester compound may readily be converted to a corresponding acid by hydrolysis, conveniently in aqueous alkali at ambient temperature.

Salts of the 4-carboxylic acid - and 4-hydroxamic acid - cinnolines are readily prepared by adding a solution of the appropriate inorganic compound.

Compounds 77-82, and 95-102 can be prepared from known substituted anilines, or substituted anilines that are readily prepared by conventional procedures. Compound 83 can be prepared by treating the 8-(chloromethyl) species with potassium cyanide in dimethylformamide at 80-120°C. The 8-chloromethyl species can be prepared by treatment of Compound 50 with a chlorinating agent such as N-chlorosuccinimide or sulfuryl chloride in an appropriate solvent such as carbon tetrachloride or dichloroethane in the presence of a free radical initiator such as benzoyl peroxide or azo-isobisbutyronitrile (AIBN) at the boiling point of the solvent used. Compound 84 can be prepared by hydrolysis of Compound 83 with sulfuric acid-water (1:1 v/v) at 95-100°C for 24-48 hours; re-esterification as described in Example 54 gives Compound 85. Compound 86 can be prepared by treating the 8-(chloromethyl) species with sodium mercaptide in hot dimethylformamide. Compound 87 can be prepared by oxidizing Compound 86 with aqueous potassium permanganate. Compound 88 can be prepared by treating the 8-(chloromethyl) species with sodium methoxide in boiling methanol. Compound 89 can be prepared by treating the 8-(chloromethyl) species with sodium phenoxide in dimethylformamide at 80°C. Compound 90 can be prepared by treating compound 50 with two equivalents of N-bromosuccinimide in an appropriate solvent such as carbon tetrachloride or dichloroethane in the presence of a free radical initiator such as benzoyl peroxide at the boiling point of the solvent to produce methyl 8-(dibromomethyl)cinnoline-4-carboxylate, which

upon treatment with silver nitrate in aqueous dioxane gives methyl 8-formylcinnoline-4-carboxylate, which is converted to 90 by treating it with diethylaminosulfur trifluoride in a freon solvent, analogous to the procedure of W. J. Middleton, Journal of Organic Chemistry, volume 40, pages 574-578 (1975). Compounds 91, 92, 93 and 94 can be prepared from Compound 57 by procedures analogous to those described above for the preparation of Compounds 83-87. Compound 103 can be prepared from 3,5-dimethylanthranilic acid by the procedures described in Example 1. Compound 104 can be prepared from 3,4-dimethylaniline, and Compound 105 can be prepared from 3,5-dimethylaniline, by the procedures described in Examples 53 and 54 for preparing compound 54 from 2-ethylaniline. Compounds 106 to 123 can be prepared by analogous procedures.

The following examples describe the preparation, isolation and physical properties of typical individual species of the compounds of Formula I, in particular instances. In each case, the identity of each product, and each of any intermediate involved, was confirmed by appropriate chemical and spectral analyses.

Example 1 - Cinnoline-4-carboxylic Acid (1)

Jacobs et al., a solid, m.p.: 196°C (with decomposition).

The diisopropylamine and bicyclohexylamine salts of 1 were prepared by mixing, in each case, an excess of the amine with 1, then distilling the excess amine.

Example 2 - Potassium Cinnoline-4-carboxylate (2)

5.10g of (1) was added over 30 minutes to a vigorously stirred solution of 2.02g of potassium carbonate in 25ml of water, at ambient temperature. The resulting mixture was stirred for one hour at ambient temperature, then treated with charcoal, and after filtration the water was evaporated. The residue was azeotropically distilled with toluene and dried at 70°C under reduced pressure, to give 2, as a light tan powder, m.p.: above 280°C.

Example 3 - Ethyl Cinnoline-4-carboxylate (3)

Jacobs et al., a solid, m.p.: 48.5-49.5°C.

Example 4 - Methyl Cinnoline-4-carboxylate (4)

A solution of 0.64g of oxalyl chloride in 5ml of dry toluene was added over 5 minutes to a stirred suspension of 0.98g of 2 in 10ml of dry toluene, at 0°C. The resulting mixture was stirred for 20 minutes at 0°C, for 30 minutes at ambient temperature, and for one hour at 80°C. The mixture was cooled and added in portions over 5 minutes to a stirred ice cold mixture of 0.4g of pyridine, 0.32g of methanol and 10ml of dry toluene. The resulting mixture was held overnight at ambient temperature, then stripped in a rotary evaporator, and the residue was chromatographed over silica gel, using 5% ethyl acetate/methylene chloride mixture as eluent, to give 4, as yellow crystals, m.p.: 57-58°C.

Example 4A - Methyl cinnoline-4-carboxylate (4)-alternative preparation

Small-scale preparation

5g of 4-(trichloromethyl)cinnoline (4A) was slowly added to 5ml of stirred concentrated (95%) sulfuric acid. The mixture was stirred for 48 hours at about 135°C. By-product hydrogen chloride was removed overhead as it formed. The mixture was cooled to ambient temperature and 50ml of anhydrous methanol was added with stirring, after which the mixture was stirred and refluxed for 48 hours. Then approximately 80% of the remaining methanol was evaporated under reduced pressure, the residue was mixed with 100ml of ether, and the mixture was stirred vigorously while 50ml of a saturated aqueous solution of sodium bicarbonate was slowly added. The liquid phases were separated, the ether phase was washed with water, dried ($MgSO_4$) and stripped of ether. The residue was purified by chromatography over silica gel, using a 1:9 v:v mixture of ethyl acetate and methylene chloride as eluent, to give 2.3g (61%) yield of 4, obtained as a solid.

Large-scale preparation

74.3g of (4A) was added slowly (over 20 minutes) to 85ml of stirred concentrated sulphuric acid. The resulting mixture was stirred at 125-130°C for 10 hours, by-product hydrogen chloride

being removed overhead as it formed. Then the mixture was cooled to 20°C and stirred and held at that temperature while 200ml of anhydrous methanol was slowly added (over 30 minutes). The resulting mixture was stirred at 70-80°C for 5 hours and then mixed with 1.7 litres of ice water. The resulting mixture was extracted with ethyl acetate, the extract was washed with 5% aqueous sodium bicarbonate solution, dried ($MgSO_4$) and stripped of solvent to give 45.7g (81% yield) of 4.

Example 4B - Preparation of (4), alternate work-up

331.7g of 4A was added over one hour to stirred 240ml of concentrated sulfuric acid. The temperature of the mixture rose to 75°C, then the stirred mixture was slowly heated (over one hour) to 125°C, and stirred at 125-130°C for 24 hours, by-product hydrogen chloride being removed overhead as it formed. The resulting mixture was cooled to 20°C and 300ml of methanol was slowly added (over 3 hours) to the stirred mixture, which heated to 50°C. The mixture was stirred at 60-70°C for 5 hours, held at ambient temperature overnight, then poured into a stirred mixture of 1.7 litres of ice water and 1.5 litres of methylene chloride. The resulting mixture was filtered through Celite (trade mark), which then was washed twice with 500ml portions of methylene chloride, the washings being added to the filtrate. The two liquid phases in the filtrate were separated. The water phase was washed twice with one-litre portions of methylene chloride, the washings being added to the separated methylene chloride solution of the ester. The combined methylene chloride phase was washed with 500ml of 5% aqueous sodium bicarbonate solution, dried ($MgSO_4$) and the solvent was evaporated to give 210g (83.4% yield) of 4.

Example 5 - n-Propyl Cinnoline-4-carboxylate (5)

A suspension of 870mg of 1, and 850mg of carbonyldiimidazole (CDII) in 30ml of tetrahydrofuran (THF) was stirred at ambient temperature until $CO_2$ evolution ceased (about 30 minutes). After being stirred for 2 hours, the resulting mixture was treated with 320mg of dry n-propanol and 3 drops of

a 0.1 M solution of sodium imidazole in THF. The mixture was held at ambient temperature overnight, then the solvent was evaporated and the residue was partitioned between water and ether. The ethereal extract was washed with saturated sodium bicarbonate solution, then with brine, then dried ($MgSO_4$) and stripped in a rotary evaporator. The residue was chromatographed over silica gel, using a 1:1 v:v mixture of cyclohexane and ethyl acetate as eluent, to give 5, as a yellow oil.

Example 6-9

By the procedures described in Example 4, the following esters of cinnoline-4-carboxylic acid were prepared from the corresponding alcohols:

n-butyl ester (6), as a yellow oil;
sec-butyl ester (7), as a yellow oil;
tert-butyl ester (8), as a solid, m.p.: 76-77°C;
benzyl ester (9), as yellow crystals, m.p.: 78-79°C.

Examples 10-13

By the procedures described in Example 4, the following esters of cinnoline-4-carboxylic acid were prepared from the corresponding phenols:

phenyl ester (10), as yellow crystals, m.p.: 116-117°C;
4-methylphenyl ester (11), as a yellow solid, m.p.: 102-103°C;
3-methylphenyl ester (12), as yellow crystals, m.p.:86-88°C;
4-chlorophenyl ester (13), as a yellow solid, m.p.: 143-145°C.

Examples 14-26

The following esters were prepared from 1 and the corresponding phenols or alcohols by the procedures described in Example 5, except that in some cases it was not necessary to include the sodium imidazole:

4-(methylthio)phenyl ester (14), as a yellow solid, m.p.: 127°C;

3-fluorophenyl ester (15), as yellow crystals, m.p.: 92-93°C;
cyclohexyl ester (16), as yellow crystals, m.p.: 89-90°C;
2,2,2-trichloroethyl ester (17), as a yellow oil:
cyclopropylmethyl ester (18), as a yellow oil;
(benzyloxycarbonyl)methyl ester (19), as a yellow oil;
2-pyridinylmethyl ester (20), as yellow crystals, m.p.: 86°C;
2-(dimethylamino)ethyl ester (21), as yellow crystals, m.p.: not determined;
(tetrahydropyran-2-yl)methyl ester (22), as a yellow oil;
2-(diethoxy phosphinyl)methyl ester, (23), as a yellow oil;
tetrahydrofuran-3-yl ester (24), as a yellow oil;
2,3-(dihydroxy)propyl ester (25), as a viscous yellow oil;
3-pyridinyl ester oxide (26), as yellow crystals, m.p.: 153-154°C (with decomposition).

Example 27 - Cinnoline-4-carboxamide (27)

A solution of 50.0g of o-isopropenylaniline (Aldrich) in 440ml of 2M hydrochloric acid was treated with 86ml of 12M hydrochloric acid at 20°C. The resulting mixture was extracted with diethyl ether to remove a small amount of an insoluble oil. The aqueous phase was cooled to 0°C and treated with 24.5g of sodium nitrite, in portions, over 2 hours, at 0-5°C. The resulting mixture was stirred for 30 minutes at 0°C, warmed to ambient temperature, heated to 60°C, and then held at ambient temperature over a weekend. It was neutralized with sodium bicarbonate, made basic with 50% sodium hydroxide solution, and extracted with ether. The extract was washed with brine, dried ($MgSO_4$), charcoaled, filtered and stripped of solvent. The residue was recrystallized from hexane to give 4-methylcinnoline (27A), as a solid, m.p.: 70-72°C.

A solution of 1.44g of 27A in 3ml of absolute ethanol was added drop-by-drop over 3 minutes to 3.4ml of stirred absolute ethanol containing 0.9g of anhydrous hydrogen chloride, at 0°C. The resulting mixture was stirred for 30 minutes, then a

solution of 1.03g of butyl nitrite in 2ml of absolute ethanol was added drop-by-drop over 3 minutes at 0°C. The mixture was stirred for 30 minutes at 0°C, for 4 hours at ambient temperature, then stored in a refrigerator overnight. A precipitated solid was collected, washed with a small amount of water, mixed with a saturated potassium bicarbonate solution, and filtered. Recrystallization from water gave 4-(hydroximinomethyl)cinnoline (27B), as a solid, m.p.: 223°C (with decomposition).

A stirred solution of 0.173g of 27B in 5ml of acetic anhydride was refluxed overnight under nitrogen. The mixture was stripped under reduced pressure, and the residue was recrystallized from hexane to give cinnoline-4-carbonitrile (27C), as a yellow crystalline solid, m.p.: 140°C.

0.76g of hydroxylamine hydrochloride was added to a stirred solution of 0.72g of 85% aqueous potassium hydroxide in 25ml of absolute ethanol at ambient temperature. The mixture was stirred at ambient temperature for 20 minutes, then 1.55g of 27C was added and the mixture was heated at reflux for 3 hours, held overnight at ambient temperature, and filtered. The filtrate was stripped of solvent to give a dark oil, from which a yellow solid precipitated on standing. The solid was separated, and stirred in hot ethyl acetate. The resulting mixture was filtered, the solvent was stripped from the filtrate and the residue was recrystallized from ethanol to give 27, as a yellows solid, m.p.: 223°C.

Example 28 - Cinnoline-4-hydroxamic acid (28)

A solution of 0.067g of sodium metal in 2.5ml of dry methanol was added to a solution of 0.20g of hydroxylamine hydrochloride in 5ml of dry methanol, at 10-20°C. The mixture then was treated with 0.50g of 4 and then with a solution of 0.061g of sodium metal in 2.4ml of dry methanol, and the resulting mixture was stirred overnight at ambient temperature. This mixture then was neutralized with 3M hydrochloric acid solution and filtered. The filtrate was stripped in a rotary evaporator and the residue was recrystallized from water to give

28, as tan crystals, m.p.: 165°C (with decomposition).

Example 29 - Potassium salt (29) of 28

0.19g of 28 was added to a slurry of 0.07g of potassium carbonate in 10ml of methanol at ambient temperature. The resulting mixture was stirred at ambient temperature for 2 hours, then stripped to dryness to give 29, as a solid, m.p.: 191-192°C (with decomposition).

Example 30 - Hydrazide of 1

2.0g of 1 was added to a solution of 1.87g of CDII in 250ml of anhydrous THF in an argon atmosphere, the resulting mixture was stirred at ambient temperature for 4 hours, 0.7g of hydrazine hydrate (as an 85% solution in water) was added over one minute to the stirred mixture. The mixture was stirred for a further 48 hours, then was stripped of solvent. The residue was dried by mixing it with 75ml of absolute alcohol, then stripping the alcohol. The resulting residue was taken up in ethyl acetate, the solution was cooled and filtered to give (30), as a yellow solid, m.p.: 172-174°C.

Example 31 - The 2-N-oxide (31) of 8

0.2g of m-chloroperoxybenzoic acid (MCPBA) was added to a cold solution of 0.2g of 8 in 20ml of methylene chloride. The resulting mixture was held in a refrigerator (2°C) overnight, then treated with a 10% aqueous solution of sodium sulfite, washed with water, then brine, dried and stripped in a rotary evaporator. The residue was flash chromatographed on silica gel using 5% ethyl acetate/methylene dichloride mixture as eluent. Work-up gave 31, as a solid, m.p.: 146-147°C.

Example 32 - 1-(Ethoxycarbonyl)ethyl Cinnoline-4-carboxylate (32)

A solution of 0.75ml of 1,8-diazabicyclo[5.4.0]undec-7-ene (DBU) in 20ml of benzene was added to a stirred suspension of 0.87g of 1 in 30ml of benzene at ambient temperature. After one hour stirring, 1.36g of ethyl 2-bromopropionate was added and the mixture was heated at reflux for 6 hours. The mixture was cooled, diluted with 25ml of benzene, washed with water, 1M aqueous sodium bicarbonate, and brine. It then was dried

($MgSO_4$), the solvent was evaporated and the residue was chromatographed on silica gel, using a 1:1 v:v mixture of cyclohexane and ethyl acetate as eluent, to give 32, as a yellow oil.

Examples 33-37

The following esters were prepared from 1 and the corresponding alcohols by the producers described in Example 32:

cyanomethyl ester (33), as yellow crystals, m.p.: 133-134°C

2,2-(dimethoxy)ethyl ester (34), as yellows crystals, m.p.: 65-67°C

(aminocarbonyl)methyl ester (35), as yellow crystals, m.p.:205-206°C.

Tert-butoxycarbonylmethyl ester (36), as a yellow solid, m.p.: 80-81°C;

1-(methoxycarbonyl)ethyl ester (37), as a yellow solid, m.p.: 85°C.

Examples 38 and 39 - N-oxides of 4

3.3g of 80% MCPBA was added in portions (over one hour) to a stirred solution of 2.67g of 4 in 100ml of dry methylene chloride, at 0°C. The mixture then was stirred at 0°C for 2.5 hours and held in a refrigerator overnight. The excess peracid was decomposed with 10% aqueous sodium thiosulfate, the mixture was washed, successively, with water, saturated sodium bicarbonate solution and brine, dried ($MgSO_4$) and stripped of solvent. The residue was flash chromatographed on silica using a 3:97 v:v mixture of ethyl acetate and methylene chloride as eluent. On workup, two solids were obtained; the first was the 1-N-oxide (38) of 4, m.p.: 109-110°C; the second was the 2-N-oxide (39) of 4, m.p.: 111-112°C.

Example 40

The free acid (40) of 39 was prepared by adding 10ml of 25% aqueous sodium hydroxide solution to a solution of 0.40g of 39 in 20ml of methanol at 20-25°C, and stirring the resulting mixture for 20 minutes. The mixture then was acidified with hydrochloric acid, stripped of most of the methanol and extracted with ethyl acetate. The extract was stripped of

solvent and the residue was recrystallized from ethanol to give 40, as a solid, m.p.: 228°C, with decomposition.

Example 41 - Cinnoline-4-carboxylic Acid (1) - Alternative Preparation

1.23 litres of a 10.5% solution of sodium hypochlorite in water, pH=12.7, was added to a stirred solution of 50.0g of 27A in 88ml of dioxane at ambient temperature and the resulting mixture was stirred for 60 hours at ambient temperature. The precipitated solid was collected, washed with water, and dried under reduced pressure, to give 4-(trichloromethyl)cinnoline (41A), as a pale green solid, m.p.: 137°C.

14.0g of 41A was slowly dissolved in 18ml of 95% sulfuric acid. The mixture was heated at 135-140°C for 20 hours, cooled, poured onto 60g of ice and made basic with 30% aqueous sodium hydroxide. The mixture was washed with methylene chloride and filtered. The filtrate was cooled to ice-bath temperature and carefully acidified to pH = 1.5-2 with concentrated hydrochloric acid. The solid product was collected, washed with a small amount of cold water and dried under reduced pressure to give 1 as a solid identical to a commercial sample.

Example 42 - 2-(2-ethoxyethoxy)ethyl cinnoline-4-carboxylate (42)

1.07g of CDII was added to a mixture of 1.04g of 1 in 70ml of dry THF under nitrogen, the mixture was stirred at ambient temperature for 4 hours, 1.6g of 2-(ethoxyethoxy)ethanol and 0.032g of sodium methoxide were added and the mixture was stirred at ambient temperature over a weekend. Then the solvent was stripped, and the residue was dissolved in methylene chloride. The solution was washed with water, dried ($MgSO_4$) and stripped of solvent. The residue was flash-chromatographed on silica gel, using a 1:4 v:v mixture of ethyl acetate and methylene chloride as eluent, to give 42, as a yellow oil.

Examples 43-48

The following esters were prepared from 1 and the corresponding alcohols by the procedures described in Example 42:

(1,3-dioxan-5-yl) ester (43), as a yellow solid, m.p.: 131-132°C;

oxiranylmethyl ester (44), as a yellow solid, m.p.: 87-88°C;

(1,3-dioxolan-4-yl)methyl ester (45), as a yellow solid, m.p.: 75-76°C;

2-(2-methoxyethoxy)ethyl ester (46), as a yellow oil;

2-(methoxy)ethyl ester (47), as a yellow solid, m.p.: 70-71°C;

2-(ethoxy)ethyl ester (48), as a yellow oil.

Example 49 - 8-Methylcinnoline-4-carboxylic Acid (49)

A solution of 60.8g of 2-amino-3-methylbenzoic acid in 900ml of dry methanol was saturated with anhydrous hydrogen chloride at 0-10°C, then allowed to stand at ambient temperature overnight. Then it was refluxed for one hour, cooled to 5°C, resaturated with anhydrous hydrogen chloride, held overnight at ambient temperature and stripped of solvent. The residue was made neutral with saturated aqueous sodium bicarbonate solution, and extracted with methylene chloride. The extract phase was washed with saturated aqueous sodium bicarbonate solution, dried ($MgSO_4$), filtered and stripped of solvent. The residue was distilled in a kugelrohr apparatus to give methyl 2-amino-3-methylbenzoate (49A), b.p.: 100-105°C (1 Torr; $1.01x10^5$Pa).

130ml of a 3M solution of methyl magnesium chloride in 130ml of THF was added drop-by-drop to a stirred solution of 11g of 49A in 70ml of dry ether at 0-5°C in a nitrogen atmosphere. The resulting mixture was allowed to warm to ambient temperature and stirred at reflux (40°C) for 5 hours and held overnight at ambient temperature. The mixture then was quenched in a mixture of 48g of ammonium chloride and 350g of ice. 30ml of 3M hydrochloric acid was added, the solvents were evaporated, and the residue was extracted with ether. The extract phase was washed with water, then brine, dried ($K_2CO_3$), filtered and

stripped of solvent. The residue was recrystallized from hexane to give (2-amino-3-methylphenyl)dimethylcarbinol (49B), as colourless needles, m.p. 78-79°C.

49B was dissolved in 60ml of toluene containing 5mg of iodine, and the mixture was refluxed overnight. The solvent was evaporated and the residue was distilled in a short-path bantumware apparatus to give 2-(2-amino-3-methylphenyl)propene (49C), as a colourless oil, b.p.: 82-85°C (2 Torr; $2.02x10^5$Pa).

6.9g of 49C was added to 55ml of 2M hydrochloric acid at 10°C. Then 11ml of 12M hydrochloric acid was added, the mixture was cooled to 0°C, treated with 3.1g of sodium nitrite, in portions over a two-hour period. The mixture was allowed to warm slowly to ambient temperature, then was heated to 50°C and left to stand over a weekend at ambient temperature. The mixture was made basic with solid sodium carbonate, and extracted with ether. The extract phase was washed with water, then brine, dried ($MgSO_4$), filtered and stripped of solvent. The residue was flash chromatographed over silica gel, using a 2:3 v:v mixture of ethyl acetate and methylene chloride as eluent. The solvent was evaporated to give 4,8-dimethylcinnoline (49D), as a solid, m.p.: 89-90°C.

45ml of commercial bleach (about 10% sodium hypochlorite, pH=12) was added to a stirred solution of 1.9g of 49D in 5ml of dioxane, at ambient temperature. The mixture was stirred at ambient temperature for 2 hours, 3ml of dioxane was added and the mixture was stirred for 4½ days at ambient temperature. Then the mixture was diluted with water and filtered. The solid was washed with water and dissolved in methylene chloride. The solution was dried ($MgSO_4$), filtered and the solvent was evaporated to give 8-methyl-4-(trichloromethyl)cinnoline (49E), as a yellow solid, m.p.: 135-137°C, with decomposition.

1.25g of 49E was slowly added (over 5 minutes) to 2ml of concentrated sulfuric acid. The resulting solution was stirred at 135-140°C overnight, then quenched on 6g of ice. The mixture was made basic with 50% aqueous sodium hydroxide solution, and extracted with methylene chloride and ethyl acetate. The

aqueous phase was carefully acidified with 12M hydrochloric acid, and filtered. The solid was dried by azeotroping with methanol and toluene. The dried solid was dissolved in methanol, the solution was filtered, then stripped of solvent, to give 49, as a solid, m.p.: 181-182°C, with decomposition.

Example 50 - Methyl 8-methylcinnoline-4-carboxylate (50)

Small scale preparation

A solution of 0.65g of 49 in 20 ml of dry THF was refluxed in a nitrogen atmosphere, then cooled to ambient temperature and 0.62g of CDII was added. The mixture was stirred for 7 hours at ambient temperature, then a suspension of 0.1 equivalent of sodium methoxide in 2 equivalents of methanol was added and the mixture was stirred in a nitrogen atmosphere overnight. The THF was evaporated, the residue was diluted with water and extracted with methylene chloride. The extract phase was washed with saturated sodium bicarbonate solution, then brine, dried ($MgSO_4$), filtered and stripped of solvent to give 50, as a solid, m.p.: 90-91°C.

Large scale preparation

261.5g of 8-methyl-4-(trichloromethyl)cinnoline (49E), prepared by the procedures described above in Example 49, was added in portions over one hour to stirred 250ml of concentrated sulfuric acid, originally at ambient temperature. The mixture warmed to 78°C. The mixture then was stirred at 130-140°C for 24 hours, then was cooled to 15°C and stirred while 300ml of methanol was slowly added, the temperature of the mixture being held at 15-30°C. The mixture then was stirred at 60-70°C for 6 hours, and at ambient temperature over a weekend. Then the mixture was mixed with a mixture of 2.5 litres of ice water and 1.5 litres of methylene chloride. The resulting mixture was filtered, the methylene chloride phase was separated, the aqueous phase was extracted three times with 500ml of methylene chloride, all of the methylene chloride solutions were combined, washed with 5% aqueous sodium bicarbonate solution, dried

($MgSO_4$) and stripped of solvent. The residue was recrystallized from ether to give 158.1g of 50, as a solid.

Example 51 - 6-Methylcinnoline-4-carboxylic acid (51)

51 was prepared, as a tan solid, m.p.: 179-180°C, from 2-amino-5-methylbenzoic acid, by the respective procedures described in Example 49 for preparing 49 from 2-amino-3-methylbenzoic acid: via methyl 2-amino-5-methylbenzoate (a solid, m.p.: 62-63°C), (2-amino-5-methylphenyl)dimethylcarbinol (an oil, b.p.: 100°C/0.1 Torr; $1.01x10^4$Pa), 2-(2-amino-5-methylphenyl)propene (a liquid, b.p.: 87-90°C/0.5 Torr; $5.05x10^4$Pa), 4,6-dimethylcinnoline (a solid, m.p.: 75-76°C), and 6-methyl-4-(trichloromethyl)cinnoline (green crystals, m.p.: 132-133°C, with decomposition).

Example 52

Methyl 6-methylcinnoline-4-carboxylate (52), was prepared, as a yellow solid, m.p.: 73-74°C, from 51 by the procedures described in Example 50 for preparing 50 from 49.

Example 53 - 8-Ethylcinnoline-4-carboxylic Acid (53)

97.0g of 2-ethylaniline was mixed with 200ml of 12M hydrochloric acid, then 400g of crushed ice was added and the mixture was cooled to -10°C. Then a solution of 56.4g of sodium nitrite in 135ml of water was added to the stirred mixture, over a period of about 35 minutes, at -10°C to 2°C, followed by 3g of urea. The mixture was stirred for 10 minutes, then was filtered through Celite (trade mark). The filtrate was added in portions to a stirred slurry of 1600ml of ethanol, 240ml of water, 128ml of diethyl malonate and 150g of anhydrous sodium acetate, at 0-3°C. The mixture was stirred and allowed to warm to ambient temperature (four hours), stirred at ambient temperature for 18 hours, then extracted with methylene chloride. The methylene chloride and ethanol were evaporated from the extract phase. The residue was dissolved in methylene chloride, the solution was washed with water, then brine, dried ($MgSO_4$), filtered, and the solvent was evaporated. The residue was stripped in a

kugelrohr apparatus to 100°C (0.5 Torr; $5.05x10^4$Pa). The residue was crystallized from ethanol/pentane, then recrystallized from petroleum ether to give diethyl ((2-ethylphenyl)hydrazono)malonate (53A), as a yellow solid, m.p.: 33-34°C.

48ml of 2M aqueous sodium hydroxide solution was added over 15 minutes to a stirred, refluxing mixture of 22.5g of 53A in 48ml of ethanol. A further 192ml of 1M aqueous sodium hydroxide solution was added over 20 minutes to the gently refluxing mixture. Then the mixture was filtered through Celite (trade mark), and the filtrate was poured into a stirred mixture of 30ml of concentrated hydrochloric acid, 90ml of water and about 100g of ice. The mixture was filtered. The solid was washed with water, dried under reduced pressure, and dissolved in methylene chloride. The solution was dried ($MgSO_4$), filtered and stripped of solvent under reduced pressure. The residue was recrystallized for ethanol/water, and dissolved in methylene chloride. The solution was dried ($MgSO_4$) and the solvent was evaporated to give ((2-ethylphenyl)hydrazono)malonic acid (53B), as a solid, m.p.: 135-136°C (with decomposition).

One equivalent of thionyl chloride was added drop-by-drop (over two minutes) to a stirred suspension of 5.58g of 53B in 30ml of 1,2-dichloroethane and 3 drops of dry dimethylformamide (DMF), under nitrogen at ambient temperature. The mixture was allowed to stand overnight at ambient temperature, then refluxed for 2.5 hours, cooled to 0°C and filtered. The solid was washed with pentane, dried and recrystallized from cyclohexane to give ((2-ethylphenyl)hydrazono)malonyl dichloride (53C), as a yellow solid, m.p.: 140-141°C (with decomposition). 53C also was prepared by treating 53B with phosphorus trichloride in chloroform.

16.4g of titanium tetrachloride was added to a stirred solution of 22.5g of 53C in 160ml of chlorobenzene, under nitrogen, at 20°C. The mixture was heated at 90-100°C for 5 hours and let stand over a weekend at ambient temperature. Then

20ml of a solution of 26.4g of sodium hydroxide in 330ml of water was added, drop-by-drop, and the mixture was stirred well and stripped of solvent to form a mush. The remainder of the sodium hydroxide solution was added, and the mixture was stirred and filtered through Celite (trade mark). The filtrate was acidified with 12M hydrochloric acid and filtered. The solid was dried under reduced pressure and dissolved in 150ml of 2M aqueous ammonium hydroxide solution. The solution was filtered. The filtrate was acidified with 12M hydrochloric acid and filtered. The solid was washed with water, dried under reduced pressure an dissolved in warm ethanol. The solution was filtered and the solvent was evaporated from the filtrate, to give 8-methyl-4-hydroxy-cinnoline-3-carboxylic acid (53D), as a solid, m.p.: 230°C (with decomposition).

A mixture of 0.7g of 53D and 2.8g of benzophenone was heated at 180-210°C until no more gas evolved (30 minutes). The mixture was cooled to 0°C, diluted with ether and filtered. The solid was slurried with hot 5M aqueous sodium hydroxide solution and filtered. The filtrate was acidified with 12M hydrochloric acid, allowed to cool and stand overnight at ambient temperature, and filtered. The resulting solid was dried under reduced pressure, and flash chromatographed on silica gel, using a 1:1 v:v mixture of ethyl acetate and methylene chloride as eluent, to give 8-ethyl-4-hydroxycinnoline (53E), as a solid, m.p.: 160-161°C.

4.0g of 53E was added in portions to stirred 20ml of phosphorus oxychloride at 29-32°C. The mixture was stirred for 15 minutes, quenched in ice water, and adjusted to pH 5-6 with sodium bicarbonate and saturated aqueous potassium bicarbonate solution, and extracted with ether. The extract phase was washed with brine, dried ($MgSO_4$), filtered and stripped of solvent. The unstable residue was immediately dissolved in 60ml of dry dimethylformamide. 3.1g of sodium p-toluenesulfinate was added to the stirred solution at 0°C under nitrogen, then the stirred mixture was allowed to warm slowly to ambient

temperature and stirred overnight. 200mg more of the sodium salt was added, the mixture was stirred vigorously at 35°C for 3 hours, and another 300mg of the sodium salt was added. The mixture was stirred at 35°C for 7 hours, then over a weekend at ambient temperature, poured into 400ml of water, and extracted with ethyl acetate. The extract phase was washed with water, then brine, dried ($MgSO_4$), filtered and stripped of solvent, and the residue was flash chromatographed over silica gel, using a 1:1 v:v mixture of ethyl acetate and hexane as eluent, to give 4(p-toluenesulfonyl)-8-ethylcinnoline (53F), as a solid, m.p.: 133-134°C.

1.0g of potassium cyanide was added to a stirred solution of 4.4g of 53F in 60ml of dry DMF at ambient temperature, under nitrogen. The mixture was stirred overnight at ambient temperature, under nitrogen, then 200mg potassium cyanide was added and the mixture was stirred for 5 hours at ambient temperature. The mixture was quenched in water and filtered. The solid was dissolved in ethyl acetate, the solution was washed with water, dried ($MgSO_4$), filtered and stripped of solvent, to give 4-cyano-8-ethylcinnoline (53G), as a yellow soled, m.p.: 102-104°C, with decomposition.

1.4g of 53G was dissolved in 60ml of 50% aqueous sulfuric acid and the mixture was stirred at 90-100°C for 32 hours. The mixture was cooled and made basic with 50% aqueous potassium hydroxide solution, then filtered. The filtrate was extracted with ether. The aqueous phase was acidified with 12M hydrochloric acid and filtered. The resulting solid was dissolved in ethyl acetate, the solution was dried and stripped of solvent to give 53, as a solid, m.p.: 175-176°C (with decomposition).

<u>Example 54</u> - Methyl 8-ethylcinnoline-4-carboxylate (54)

A solution of 1.1g of 53, 70ml of dry THF and 0.98g of CDII was stirred under nitrogen at ambient temperature until no more gas evolved (4 hours), 0.35g of methanol and 0.03g of sodium acetate were added and the mixture was stirred at ambient

temperature for 3 hours. The THF was evaporated, the residue was dissolved in methylene chloride, the solution was washed with water, dried ($MgSO_4$) and stripped of solvent. The residue was recrystallized from hexane to give 54, as a yellow solid, m.p.: 59-60°C.

Examples 55 and 56

2,2,2-Trichloroethyl 8-methylcinnoline-4-carboxylate (55) was prepared, as a yellow solid, m.p.: 116-118°C, with decomposition, and ethyl 8-methylcinnoline-4-carboxylate (56) was prepared, as a yellow solid, m.p.: 65-66°C, by treating 49 with CDII and the appropriate alcohols, according to the procedures described in Example 50 for preparing 50 from 49.

Example 57

Methyl 8-isopropylcinnoline-4-carboxylate (57), was prepared, as a yellow oil, form 2-isopropylaniline by the procedures described in Example 53 and 54 for preparing 54 from 2-ethylaniline.

Example 58 - Methyl 8-(fluoromethyl)cinnoline-4-carboxylate (58)

To 4.3g of 50 in 1900ml of carbon tetrachloride a few milligrams of benzoyl peroxide was added, followed by 3.2g of N-bromosuccinimide and the stirred mixture was refluxed and irradiated with a 200-watt tungsten lamp for 6 hours. The resulting mixture cooled, washed with water, then with very dilute aqueous sodium bicarbonate solution, dried ($MgSO_4$) and stripped of solvent. The residue (methyl 8-(bromomethyl)cinnoline-4-carboxylate) (58A) was dissolved in 80ml of acetone, 2.11g of lithium chloride was added, and the mixture was stirred at ambient temperature over a weekend. The mixture was stripped of solvent and the residue was dissolved in methylene chloride. The solution was washed with water, dried ($MgSO_4$) and stripped of solvent. The residue was flash chromatographed over silica gel, first using methylene chloride as eluent, then a 1:99 v:v mixture of ethyl acetate and methylene chloride as eluent, to give methyl

8-(chloromethyl)cinnoline-4-carboxylate (58B), as a solid, m.p.: 111-112°C (with decomposition).

A mixture of 0.35g of 58B, 3.0g of Amberlyst A26 (trade mark) fluoride resin and 5ml of benzene was stirred vigorously at mild reflux in a nitrogen atmosphere overnight. The resulting mixture was filtered, and the filtrate was stripped of solvent. The residue was chromatographed over silica gel, using a 1:99 v:v mixture of ethyl acetate and methylene chloride as eluent, to give 58, as a yellow solid, m.p.: 104-105°C.

Example 59 - Methyl 6,7-dimethylcinnoline-4-carboxylate (59)

95ml of acetic anhydride was added to a stirred solution of 3,4-dimethylaniline in 300ml of glacial acetic acid at such a rate that the temperature of the mixture slowly rose to 60°C. The resulting mixture was stirred while the temperature dropped to ambient temperature, then evaporated to dryness. A mixture of water and ice was added to the residue, followed by methylene chloride. The resulting mixture was stirred and treated with solid sodium bicarbonate until neutral. The organic phase was separated, and dried ($MgSO_4$), and the solvent was evaporated to give 3,4-dimethylacetanilide (59A).

105ml of acetyl chloride was added to a solution of 130g of 59A in 1040ml of carbon disulfide, then 410g of aluminum chloride was slowly added to the stirred mixture. The mixture was stirred at reflux for 1.5 hours, cooled and the carbon disulfide phase was decanted. The remainder was poured onto ice, and the resulting precipitate was collected, washed with water and dried to give 2-acetyl-4,5-dimethylacetanilide (59B).

A mixture of 44g of 59B and 300ml of concentrated hydrochloric acid was refluxed for 30 minutes, then cooled in an ice bath and filtered. The collected solids were partitioned between methylene chloride and a saturated aqueous solution of sodium bicarbonate. The methylene chloride phase was separated and dried ($MgSO_4$), and the solvent was evaporated under reduced

pressure. The residue was recrystallized from a 2:1 v:v mixture of hexane and ethyl acetate to give 2-amino-4,5-dimethyl-acetophenone (59C).

10.5g of 50C was dissolved in 30ml of concentrated hydrochloric acid and was kept cold with an ice bath, then 50ml of water was added and the resulting mixture was treated at about 0°C with sodium nitrite until the diazotation was complete by iodine-starch reaction. The resulting mixture was poured onto an ice-cold mixture of 50g of sodium acetate, 150ml of water, 50g of ice and 50ml of methylene chloride, and the mixture was stirred overnight, spontaneously warming to ambient temperature. The resulting mixture was filtered, the collected solid was washed with methylene chloride, then water, and dried in a vacuum oven. The product was recrystallized from ethanol to give 6,7-dimethyl-4-hydroxycinnoline (59D), as orange crystals, m.p.: above 260°C. Further 59D was obtained by adding petroleum ether to the mother liquor from the recrystallization.

59D was converted to 59, obtained as a yellow solid, m.p.: 119-120°C (with decomposition), by treating 59D according to the procedures described in Example 53 for converting 53E to 53.

<u>Examples 60</u> - 7,8-Dimethylcinnoline-4-carboxylic acid (60)

60 was prepared, as a gold-coloured solid, m.p.: 189°C (with decomposition) from 7,8-dimethyl-4-hydroxycinnoline (60A), according to the procedures described in Example 53 for preparing 53 from 53E. 60A was prepared as follows:

22.3ml of concentrated hydrochloric acid was added drop-by-drop to a stirred suspension of 2-amino-3,4-dimethylacetophenone (A. Brandstrom and S. A. I. Carlson, Acta Chemica Scandinavica, volume 21, pages 983-992 (1967)), 100ml of water and 100g of ice at 0°C. The resulting mixture was stirred at 0°C for 15 minutes, then a solution of 7.8g of sodium nitrite in 60ml of water was added drop-by-drop over 2 minutes, at 0°C. The mixture was stirred for 40 minutes at 0°C, then 200ml of ice-cold methylene chloride and 200ml of an ice-cold aqueous solution containing 81.5g of sodium acetate were added. The

mixture was stirred for 6 hours at 0°C, allowed to warm gradually to ambient temperature, then filtered. The collected solid was dried by azeotroping with toluene and recrystallized from ethanol-hexane to give 60A, as a tan solid, m.p.: above 260°C.

Example 61 - Methyl 7-methylcinnoline-4-carboxylate (61)

7-Methyl-4-hydroxycinnoline (61A) was prepared from 3-methylaniline by the procedures described in Example 53 for preparing 53E from 2-ethylaniline. 61A was converted to 61, obtained as a yellow solid, m.p.: 114-115°C, by the procedures described in Example 53 and 54 for preparing 53 from 53E.

Example 62 - 6,7-Dimethylcinnoline-4-carboxylic acid (62)

0.95g of 6,7-dimethyl-4-cinnolinecarbonitrile (obtained as an intermediate in the preparation of 59) was mixed with 50ml of a 50% aqueous solution of sulfuric acid, and the mixture was stirred at 90-100°C overnight. The resulting mixture was made basic with 50% aqueous sodium hydroxide solution and filtered. The filtrate was extracted with ether. The remaining aqueous phase was acidified with 12M hydrochloric acid and extracted with ethyl acetate. The extract was dried ($MgSO_4$) and stripped of solvent to give 62, as a yellow solid, m.p.: 204-205°C (with decomposition).

Examples 63-70

The following esters were prepared from 49 and the corresponding alcohols by the procedures described in Example 5 for preparing 5 from 1:

2-pyridinylmethyl ester (63), as a yellow solid, m.p.: 132-133°C;

2,2-(dimethoxy)ethyl ester (64), as a yellow solid, m.p.: 72-73°C;

1,3-dioxolan-4-ylmethyl ester (65), as a yellow solid, m.p.: 100-101°C;

1,3-dioxan-5-yl ester (66), as a yellow solid, m.p.: 138-139°C;

2-(2-ethoxyethoxy)ethyl ester (67), as a yellow oil;

oxiranylmethyl ester (68), as a yellow solid, m.p.: 65-67°C;

2-(2-methoxyethoxy)ethyl ester (69), as a yellow solid, m.p.: 42-43°C;

2-(methoxy)ethyl ester (70), as a yellow solid, m.p.: 75-76°C.

Examples 71 and 72

By similar procedures, the 2,2-(dimethoxy)ethyl esters of 53 and 57 were prepared, as a yellow solid (71), m.p.: 33-34°C, and a yellow liquid (72) respectively.

Example 73

The 2-(ethoxy)ethyl ester of 49 was prepared as a yellow oil (73) from 49 and 2-(ethoxy)ethanol by the procedures described in Example 42 for preparing 42 from 1.

Example 74

The methyl ester of 60 was prepared as yellow needles (74), m.p.: 95-96°C, from 60 and methanol, according to the procedures described in Example 45 for preparing 45 from 1.

Example 75

The 2-N-oxide of 50 was prepared, as a yellow solid (75), m.p.: 145-147°C, by oxidation of 50, by the procedure described in Example 31 for preparing 31 from 8.

Example 76 - Carboxymethyl Cinnoline-4-carboxylate Isopropylamine Salt (76)

A solution of 2.4g of 19 in 150ml of methanol containing 0.75g of 5% palladium-on-carbon catalyst was hydrogenated (40psig; $2.7x10^5$Pa) for 19 hours. The resulting mixture was filtered, and stripped of solvent. The residue was dissolved in an excess of isopropylamine, then the amine was evaporated from the solution under reduced pressure, to give 76, as tan crystals, m.p.: 173°C (with decomposition).

Compounds of Formula I have been found to adversely affect the growth of some plants, many of which are commonly considered as weeds, and therefore to be useful for controlling the growth

of such unwanted plants. Compounds of Formula I have been found to have selectivity with respect to some crop plants - i.e., they control weeds at dosages at which they do not significantly harm the crop plants. While compounds of Formula I appear to have some activity when applied preemergence or preplant incorporated (applied to the soil before the seeds have sprouted), most appear to be more effective when applied postemergence (applied to the foliage of the growing plant).

Examples of Activity with Respect to Plants

Primary Tests

In the following examples, the species of plants that were tested were:

Barnyardgrass (watergrass) - Echinochloa crus-galli
Large crabgrass - Digitaria sanguinalis
Downy brome - Bromus tectorum
Yellow foxtail - Setaria lutescens
Redroot pigweed - Amaranthus retroflexus
Sicklepod - Cassia obtusifolia
Velvetleaf - Abutilon theophrasti
Garden cress - Lepidium sativum
Johnson grass - Sorghum halepense
Morningglory - Ipomoea sp.

Test Procedures

The preemergence (soil) herbicidal activity of compounds of Formula I was evaluated by planting seeds of barnyardgrass, garden cress, downy brome, velvetleaf, yellow foxtail, and sicklepod or morningglory in test tubes, nominally measuring 25 x 200 millimeters, filled about three-quarters full of untreated soil, in each case covered on top with about 2.5 cubic centimeters of soil treated with a certain amount of the test compound. The treated soil applied to the tubes containing the barnyardgrass and cress seeds contained one milligram of the test compound per tube, and contained 0.1 milligram of the test compound per each tube containing the seeds of the other plants. The dosages were approximately 20 and 2.0 pounds of test

compound per acre, that is, approximately (22.4kg and 2.2kg per hectare) respectively. The seeds were planted on top of the treated soil and covered with about 1.5 cubic centimeters of untreated soil. The planted soil was held under controlled conditions of temperature, moisture, and light for 9 to 10 days. The amounts of germination and growth in each tube were evaluated on a 0 to 9 scale, the numeric ratings having the following meanings:

| Rating | Meaning |
|---|---|
| 9 | No living tissue |
| 8 | Plant severely damaged and expected to die |
| 7 | Plant badly damaged, but expected to live |
| 6 | Moderate damage, but complete recovery expected |
| 5 | Intermediate damage (probably unacceptable for crop plants) |
| 3-4 | Observable damage |
| 1-2 | Plant slightly affected, possibly by the chemical, possibly due to biological variability |
| 0 | No visible effect |

The postemergence (foliar) herbicidal activity of compounds of Formula I was evaluated by spraying 10-day-old large crabgrass plants, 13-day-old pigweed plants, 6-day-old johnsongrass plants, 9-day-old velvetleaf plants, 9-day-old yellow foxtail plants and eight 9-day-old sicklepod plants or 5-day-old morningglory plants to runoff with a liquid formulation of the test compound. The crabgrass and pigweed plants were sprayed with 2.4 millilitres of a 0.25% solution (about ten pounds of the test compound per acre, that is, about 11.2kg per hectare), and other plants were sprayed with 2.4 millilitres of a 0.025% solution (about one pound of the test compound per acre, that is, about 1.1kg per hectare). The sprayed plants were held under controlled conditions of temperature, moisture and light for 7 to 8 days, when the effect of the test compound was evaluated visually, the results being rated on the 0 to 9 scale described above.

Results of the preemergence and postemergence herbicidal activity tests are set forth in Table I.

TABLE I - HERBICIDAL ACTIVITY

| | Preemergence | | | | | | | Postemergence | | | | | | |
|---|---|---|---|---|---|---|---|---|---|---|---|---|---|---|
| Compound | A | B | C | D | E | F | G | H | I | J | D | E | F | G |
| 1 | 3 | 6 | 3 | 0 | 7 | 3 | - | 7 | 5 | 0 | 2 | 9 | 2 | - |
| 2 | 2 | 2 | 2 | 0 | 8 | 2 | - | 9 | 4 | 1 | 2 | 9 | 2 | - |
| 3 | 2 | 6 | 0 | 0 | 9 | 0 | - | 9 | 4 | 0 | 1 | 9 | 2 | - |
| 4 | 3 | 4 | 0 | 0 | 8 | 2 | - | 9 | 5 | 0 | 2 | 9 | 2 | - |
| 5 | 0 | 2 | 2 | 0 | 8 | 3 | - | 9 | 6 | 1 | 2 | 9 | 3 | - |
| 6 | 0 | 2 | 0 | 0 | 8 | 0 | - | 9 | 6 | 0 | 2 | 9 | 3 | - |
| 7 | 0 | 7 | 0 | 2 | 8 | 0 | - | 9 | 6 | 0 | 2 | 8 | 2 | - |
| 8 | 0 | 5 | 0 | 0 | 8 | 3 | - | 5 | 8 | 0 | 2 | 5 | 2 | - |
| 9 | 0 | 0 | 0 | 0 | 8 | 0 | - | 7 | 5 | 1 | 2 | 9 | 3 | - |
| 10 | 2 | 3 | 0 | 0 | 8 | 2 | - | 8 | 5 | 0 | 0 | 9 | 0 | - |
| 11 | 2 | 3 | 0 | 0 | 8 | 2 | - | 6 | 5 | 0 | 2 | 9 | 2 | - |
| 12 | 2 | 2 | 0 | 0 | 8 | 0 | - | 8 | 5 | 0 | 0 | 9 | 2 | - |
| 13 | 0 | 0 | 0 | 0 | 9 | 0 | - | 5 | 0 | 1 | 0 | 9 | 2 | - |
| 14 | 0 | 0 | 0 | 0 | 9 | 0 | - | 9 | 4 | 0 | 0 | 9 | 3 | - |
| 15 | 0 | 0 | 0 | 0 | 8 | 0 | - | 7 | 6 | 0 | 0 | 9 | 2 | - |

TABLE I - HERBICIDAL ACTIVITY continued

| | Preemergence | | | | | | | Postemergence | | | | | | |
|---|---|---|---|---|---|---|---|---|---|---|---|---|---|---|
| Compound | A | B | C | D | E | F | G | H | I | J | D | E | F | G |
| 16 | 0 | 0 | 0 | 0 | 8 | 0 | – | 6 | 5 | 0 | 0 | 9 | 2 | – |
| 17 | 2 | 2 | 0 | 0 | 9 | 0 | – | 7 | 5 | 0 | 0 | 9 | 3 | – |
| 18 | 2 | 4 | 0 | 0 | 8 | 0 | – | 7 | 6 | 0 | 2 | 9 | 2 | – |
| 19 | 0 | 0 | 0 | 0 | 7 | – | 2 | 8 | 6 | 0 | 0 | 9 | – | 4 |
| 20 | 0 | 0 | 0 | 0 | 7 | – | 0 | 7 | 5 | 0 | 2 | 9 | – | 3 |
| 21 | 0 | 0 | 0 | 0 | 0 | – | 0 | 9 | 7 | 3 | 2 | 9 | – | 2 |
| 22 | 3 | 3 | 2 | 2 | 8 | – | 4 | 9 | 5 | 0 | 2 | 9 | – | 3 |
| 23 | 0 | 0 | 0 | 0 | 0 | – | 0 | 6 | 6 | 0 | 0 | 9 | – | 2 |
| 24 | 0 | 6 | 0 | 0 | 0 | – | 0 | 4 | 3 | 0 | 0 | 9 | – | 4 |
| 25 | 0 | 0 | 0 | 0 | 0 | – | 0 | 4 | 2 | 3 | 0 | 8 | – | 5 |
| 26 | 0 | 0 | 0 | 0 | 8 | – | 0 | 7 | 4 | 0 | 2 | 9 | – | 5 |
| 27 | 0 | 0 | 0 | 0 | 6 | 0 | – | 5 | 5 | 0 | 0 | 9 | 3 | – |
| 28 | 0 | 0 | 0 | 0 | 8 | 0 | – | 4 | 4 | 0 | 0 | 8 | 3 | – |
| 29 | 0 | 0 | 0 | 0 | 9 | 0 | – | 5 | 4 | 0 | 0 | 9 | 0 | – |
| 30 | – | – | 0 | 0 | 3 | 0 | 0 | 5 | 4 | 0 | 0 | 9 | 4 | 5 |

TABLE I - HERBICIDAL ACTIVITY continued

| | Preemergence | | | | | | | Postemergence | | | | | | |
|---|---|---|---|---|---|---|---|---|---|---|---|---|---|---|
| Compound | A | B | C | D | E | F | G | H | I | J | D | E | F | G |
| 31 | 0 | 3 | 2 | 0 | 9 | 0 | - | 2 | 3 | 0 | 0 | 7 | 0 | - |
| 32 | 0 | 0 | 0 | 0 | 0 | 0 | - | 8 | 6 | 0 | 2 | 9 | 2 | - |
| 33 | 0 | 0 | 0 | 0 | 8 | - | 0 | 9 | 5 | 0 | 0 | 9 | - | 0 |
| 34 | 2 | 6 | 0 | 3 | 7 | - | 2 | 5 | 3 | 0 | 0 | 9 | - | 0 |
| 35 | 4 | 3 | 0 | 0 | 9 | - | 3 | 7 | 3 | 0 | 0 | 9 | - | 6 |
| 36 | 0 | 0 | 0 | 0 | 8 | - | 8 | 8 | 5 | 2 | 0 | 9 | - | 3 |
| 37 | 3 | 0 | 0 | 0 | 9 | - | 7 | 5 | 6 | 0 | 0 | 9 | - | 0 |
| 38 | 5 | 7 | - | 3 | 8 | 0 | - | 5 | 7 | 2 | 2 | 8 | 2 | - |
| 39 | 2 | 3 | 0 | 0 | 8 | 0 | - | 9 | 3 | 0 | 2 | 9 | 2 | - |
| 40 | 0 | 0 | 0 | 0 | 0 | 0 | - | 9 | 2 | 2 | 0 | 9 | 2 | - |
| 42 | - | - | 0 | 0 | 9 | 0 | 0 | 9 | 0 | 0 | 0 | 9 | 0 | 9 |
| 43 | - | - | 0 | 0 | 9 | 0 | 0 | 9 | 0 | 3 | 0 | 8 | 0 | 8 |
| 44 | - | - | 0 | 0 | 9 | 0 | 0 | 9 | 0 | 6 | 0 | 9 | 0 | 8 |
| 45 | - | - | 0 | 7 | 9 | 0 | 0 | 9 | - | 3 | 0 | 9 | 0 | 9 |
| 46 | - | - | 0 | 9 | 9 | 0 | 2 | 9 | - | 0 | 0 | 9 | 0 | 9 |

TABLE I - HERBICIDAL ACTIVITY continued

| | Preemergence | | | | | | | Postemergence | | | | | | |
|---|---|---|---|---|---|---|---|---|---|---|---|---|---|---|
| Compound | A | B | C | D | E | F | G | H | I | J | D | E | F | G |
| 47 | – | – | 0 | 0 | 8 | 0 | 0 | 9 | – | 3 | 0 | 9 | 0 | 9 |
| 48 | – | – | 0 | 0 | 9 | 0 | 4 | 9 | – | 3 | 2 | 9 | 0 | 9 |
| 49 | 0 | 6 | 0 | 2 | 6 | – | 5 | 3 | 6 | 3 | 3 | 3 | – | 4 |
| 50 | 0 | 0 | 0 | 0 | 0 | – | 0 | 7 | 2 | 2 | 0 | 9 | – | 2 |
| 51 | 0 | 0 | 0 | 0 | 0 | 0 | – | 9 | 3 | 2 | 0 | 8 | – | 2 |
| 52 | 0 | 0 | 0 | 0 | 0 | – | 0 | 9 | 2 | 2 | 0 | 8 | – | 3 |
| 53 | 0 | 7 | 0 | 3 | 6 | – | 4 | 3 | 4 | 0 | 4 | 4 | – | 2 |
| 54 | 0 | 0 | 0 | 0 | 0 | – | 0 | 6 | 6 | 2 | 2 | 6 | – | 9 |
| 55 | 0 | 2 | 0 | 0 | 3 | – | 0 | 2 | 0 | 1 | 0 | 6 | – | 2 |
| 56 | 0 | 0 | 0 | 0 | 3 | – | 3 | 4 | 9 | 2 | 4 | 6 | – | 2 |
| 57 | 0 | 3 | 0 | 0 | 0 | | 0 | 8 | 9 | 0 | 2 | 5 | | 0 |
| 58 | – | – | 0 | 0 | 6 | 0 | 0 | 5 | 0 | 2 | 0 | 9 | 0 | 3 |
| 59 | 2 | 3 | 0 | 2 | 0 | – | 3 | 6 | 5 | 2 | 3 | 7 | – | 2 |
| 60 | 2 | 3 | 0 | 0 | 0 | – | 0 | 0 | 0 | 0 | 0 | 9 | – | 0 |
| 61 | 0 | 2 | 0 | 0 | 0 | – | 0 | 3 | 2 | 0 | 0 | 7 | – | 0 |

44

TABLE I - HERBICIDAL ACTIVITY continued

| | Preemergence | | | | | | | Postemergence | | | | | | |
|---|---|---|---|---|---|---|---|---|---|---|---|---|---|---|
| Compound | A | B | C | D | E | F | G | H | I | J | D | E | F | G |
| 62 | 0 | 0 | 0 | 0 | 0 | - | 0 | 0 | 0 | 0 | 0 | 7 | - | 0 |
| 63 | 0 | 0 | 0 | 0 | 0 | - | 0 | 4 | 2 | 0 | 2 | 9 | - | 2 |
| 64 | - | - | 0 | 0 | 0 | - | 0 | 9 | 3 | 0 | 4 | 9 | 2 | 9 |
| 65 | - | - | 0 | 0 | 0 | 0 | 0 | 7 | 0 | 3 | 4 | 9 | 0 | 0 |
| 66 | - | - | 0 | 0 | 0 | 0 | 0 | 6 | 0 | 0 | 4 | 9 | 0 | 9 |
| 67 | - | - | 0 | 0 | 5 | 0 | 0 | 8 | 0 | 3 | - | 9 | 0 | 8 |
| 68 | - | - | 0 | 0 | 0 | 0 | 0 | 7 | 0 | 6 | 3 | 8 | 0 | 8 |
| 69 | - | - | 0 | 0 | 0 | 0 | 0 | 7 | 0 | 0 | 0 | 9 | 0 | 9 |
| 70 | - | - | 0 | 0 | 0 | 0 | 0 | 8 | 0 | 3 | 3 | 9 | 2 | 9 |
| 71 | - | - | 0 | 0 | 0 | 0 | 0 | 9 | 2 | 2 | 2 | 9 | 2 | 8 |
| 72 | - | - | 0 | 0 | 0 | 0 | 0 | 5 | 2 | 0 | 2 | 9 | 0 | 8 |

**TABLE I - HERBICIDAL ACTIVITY continued**

| | Preemergence | | | | | | | Postemergence | | | | | | |
|---|---|---|---|---|---|---|---|---|---|---|---|---|---|---|
| Compound | A | B | C | D | E | F | G | H | I | J | D | E | F | G |
| 73 | - | - | 0 | 0 | 0 | 0 | 0 | 8 | 0 | 0 | 3 | 9 | 2 | 9 |
| 74 | 0 | 0 | 0 | 0 | 0 | - | 0 | 3 | 4 | 0 | 0 | 7 | - | 0 |
| 75 | 0 | 0 | 0 | 0 | 0 | - | 0 | 0 | 0 | 0 | 0 | 6 | - | 5 |
| 76 | 4 | 3 | 3 | 2 | 8 | - | 3 | 9 | 4 | 0 | 0 | 9 | - | 3 |

Key:

| | | | |
|---|---|---|---|
| Barnyard grass | - A | Sicklepod | - F |
| Garden Cress | - B | Morningglory | - G |
| Downy Brome | - C | Crabgrass | - H |
| Velvetleaf | - D | Pigweed | - I |
| Yellow Foxtail | - E | Johnsongrass | - J |

Secondary Tests

In the following examples, the species of plants that were tested were:

Barnyardgrass
Downy Brome
Johnsongrass
Wild oats - *Avena fatua*
Yellow foxtail
Goose grass - *Eleusine indica*
Yellow nutsedge - *Cyperus esculentus*
Cocklebur - *Xanthum pennsylvanicum*
Morningglory
Wild mustard - *Brassica kaber*
Redroot pigweed
Sicklepod
Velvetleaf
Corn - *Zea mays*
Cotton - *Gossypium hirsutum*
Rice - *Oryza sativa*
Grain sorghum - *Sorghum vulgare*
Soybeans - *Glycine max*
Sugarbeets - *Beta vulgaris*
Wheat - *Triticum aestivum*
Nightshade - *Solarium* sp.

Test Procedures

The preemergence activity of compounds of Formula I was further determined with respect to certain species of crop plants and common species of weeds, by spraying a formulation of the test compound on soil in small pots in which seeds of the plants had been sown. The postemergence herbicidal activity of compounds of Formula I was evaluated with respect to the crop plants and weeds, by spraying a formulation of the test compound on the foliage of the young growing plants. In each series of tests, the plants were grown in pots placed in narrow trays and sprayed with the formulation. Dosages of the test compounds of

4.0 pounds/acre in some cases, and 1.0 or 0.25 pound/acre in all cases, were used that is, about 4.5, 1.1 or 0.3kg per hectare. The results of the tests were evaluated on the basis of the 0-9 scale described with respect to the earlier tests. Activity of the test compound in such case was characterized as follows:

| | Dosage (kg/ha.) | Rating (one or another) |
|---|---|---|
| Highly active | 0.3 | 8-9 |
| | 1.1 | 8-9 |
| | 4.5 | 8-9 |
| Very active | 0.3 | 6-7 |
| | 1.1 | 8-9 |
| | 4.5 | 8-9 |
| Active | 0.3 | 4-5 |
| | 1.1 | 5-7 |
| | 4.5 | 7-9 |
| Slightly active | 0.3 | 2-3 |
| | 1.1 | 3-4 |
| | 4.5 | 4-5 |
| Essentially inactive | 0.3 | 0 |
| | 1.1 | 0-3 |
| | 4.5 | 1-3 |

Compounds 1-3, 11, 13, 15-18 and 37 were tested preemergence: Compound 1 was very active with respect to yellow foxtail; active with respect to rice and cocklebur; slightly active with respect to morningglory; and essentially inactive with respect to all of the other species of test plants.

Compounds 2, 3, 11, 13, 15-18 and 37 were very active respect to yellow foxtail and essentially inactive with respect to all of the other species of the plants tested.

Compounds 1-37, 39-41, 44, 49-51, 54, 56, 57, 59-63, 69, 71, 72, 74 and 76 were tested postemergence.

With respect to yellow foxtail, Compounds 1-4, 9, 12, 15, 19-26, 28, 29, 32-36, 39, 44, 69 and 76 were highly active; Compounds 5-6, 10, 17, 18, 37, 40, 50, 51 and 72 were very

active; Compounds 7, 11, 13, 14, 16, 27, 30, 49, 63 and 71 were active; and Compounds 8 and 31 were slightly active.

With respect to cocklebur, Compounds 1-6, 9, 11, 12, 15, 17, 18, 20-23, 27, 28, 32, 33, 35, 51 and 72 were highly active; Compounds 16, 19, 34, 36, 37, 39, 44, 50, 69 and 76 were very active; Compounds 13, 21, 25, 26, 29, 30, 40, 49, 54, 56, 57, 71 and 72 were active; Compounds 7, 10, 14, 24, 61 and 63 were slightly active; and Compounds 8, 10 and 31 were essentially inactive.

With respect to morningglory, Compounds 2, 3, 4, 6, 12, 32 and 36 were highly active; Compounds 9, 17, 28, 29, 34 and 37 were very active; Compounds 5, 10, 15, 16, 18, 20, 25, 33, 39, 44, 49, 50, 54, 56, 57, 69 and 76 were active; Compounds 1, 7, 11, 13, 14, 19, 24, 50, 51, 61 and 63 were slightly active; and Compounds 7, 8, 22 and 26 were essentially inactive.

With respect to nightshade, Compound 9 was very active; Compounds 3-6, 12, 13, 15-18, 20, 33, 49, 50, 54 and 56 were active; Compounds 1, 2, 7, 11, 14, 19, 21-26, 28, 30, 32, 51 and 57 were slightly active; and Compounds 8, 10, 27, 31, 32, 38, 40 and 51 were essentially inactive.

With respect to pigweed, Compound 21 was active; Compounds 19, 22, 23, 26, 30, 39 and 62 were slightly active.

With respect to wild mustard, Compound 50 was found to be very active; Compounds 20, 33, 49, 51 and 56 were found to be active.

With respect to velvetleaf, Compounds 50 and 54 were found to be active; Compounds 22, 30, 36 and 72 were found to be slightly active.

With respect to all of the other varieties of plants, all of the compounds were essentially inactive.

The amine salts were found to have essentially the same activity as 1.

Compound 1 also was found to be very active with respect to crabgrass and field sandbur (Cenchrus incertus) when applied postemergence.

K 3436 III FF

## CLAIMS

1. A method for controlling the growth of unwanted plants at a locus, which method comprises applying to the locus an effective amount of a compound of the formula

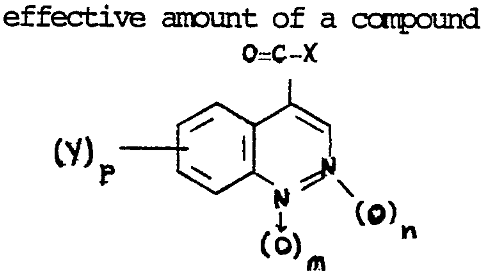

(I)

wherein m is zero or one, n is zero or one, (with the proviso that at least one of m and n is zero), p is zero, one or two, (with the proviso that when p is two, one of Y is methyl or ethyl), X is one of the moieties: $-NH_2$, $-NHNH_2$, -NHOH and -O-R, wherein R is

(a) hydrogen;

(b) alkyl of one to ten carbon atoms;

(c) alkyl of one to ten carbon atoms substituted by one of cyano, alkanoyl, carboxy, alkoxycarbonyl, aminocarbonyl, mono- and di-alkylaminocarbonyl, benzyloxycarbonyl, alkylthiocarbonyl, alkenyl and alkynyl of two to four carbon atoms, cycloalkyl of three to six carbon atoms, oxiranyl, dialkoxyphosphinyl, tetrahydrofuranyl, tetrahydropyranyl, dioxanyl, dioxolanyl, pyridinyl, pyridinyl-N-oxide, and optionally substituted phenyl;

(d) alkyl of two to ten carbon atoms substituted by one to three halogen atoms (bromine, chlorine, fluorine); one of alkylthio, alkanoyloxy, mono- and dialkylamino, moieties of the formula $-(-O-R^1-)_n-O-R^2$ (in which $R^1$ is alkylene of one to four carbon atoms, $R^2$ is hydrogen or alkyl of one to six carbon atoms and n is one, two, three or four), or one to four of hydroxy and alkoxy, with the proviso that in this substituted alkyl moiety the carbon atom bonded to the indicated oxygen atom is unsubstituted ($-CH_2-$);

(e) cycloalkyl of three to six carbon atoms;

(f) optionally substituted phenyl;

(g) pyridinyl;

(h) heterocyclyl of five or six atoms containing one or two oxygen atoms bonded only to carbon therein;

and Y is

(a) alkyl of one to ten carbon atoms;

(b) alkyl of one to ten carbon atoms substituted by one of fluorine, cyano, carboxy, alkenyl of two to four carbon atoms, benzyl, alkoxycarbonyl, alkylthio, alkylsulfinyl, alkoxy and phenoxy;

and salts of those compounds wherein R is hydrogen or X is -NHOH.

2. A method according to claim 1, wherein X represents a group of formula -O-R where R is as defined in claim 1.

3. A method according to claim 1 or 2 wherein R represents a hydrogen atom or a substituted or unsubstituted alkyl group.

4. A method according to any preceding claim wherein p is one or two and a substituent Y is bonded to the carbon atom in the 8-position.

5. A herbicidal composition comprising a compound of formula I as defined in any preceding claim, together with at least two carriers, at least one of which is a surface-active agent.

6. A method of preparing a herbicidal composition which comprises mixing a compound of formula I, as defined in any of claims 1 to 4, with at least one carrier.
7. A compound of formula I, as defined above, provided that when p and m are both 0 and X is -OR, R is not hydrogen, ethyl or di($C_{1-2}$alkyl)amino-$C_{1-2}$alkyl.
8. A process for the preparation of a compound of formula I, as defined in any of claims 1 to 4, or 7, which comprises treating a corresponding (4-trichloromethyl)cinnoline with concentrated sulphuric acid, to form a (4-carboxylic acid)cinnoline of formula I, and, when an ester of formula I is required, converting said acid to the ester, and when a compound of formula I is required in which X represents the group $-NHNH_2$, treating a said acid with hydrazine and, when a compound of formula I is required in which X represents the group -NHOH, treating a said ester with hydroxylamine; and, when a compound of formula I is required in which X is a group $-NH_2$, treating a 4-carbonitrile cinnoline compound with hydroxylamine; and converting a compound thus prepared into a salt or N-oxide thereof, or into any other compound of formula I.
9. A process according to claim 8, wherein at least three moles of concentrated sulphuric acid are used per mole of (4-trichloromethyl) cinnoline compound.
10. A process according to claim 8 or 9, wherein the (4-trichloromethyl)cinnoline compound has been prepared by treating the corresponding 4-methylcinnoline with a stoichiometric excess of sodium hypochlorite in a liquid phase comprising essentially water and a polar, water-miscible inert organic liquid as co-solvent, the pH of the liquid medium being between about 12 and about 13.5 and the concentration of the sodium hypochlorite being from about 8 to about 15 per cent by weight, based on the weight of the liquid phase.

11. A compound of formula I, as defined in any of claims 1 to 4, or 7, when produced by the process of claim 8.
12. Use of a compound of formula I, as defined in any of claims 1 to 4, in combating weed growth.
13. A compound of formula

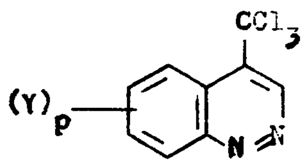

(II)

where Y and p are as defined in any of claims 1 to 4 or 7.